# EUROPÄISCHE PATENTSCHRIFT

(11) **EP 0 999 210 B1**
(45) Veröffentlichungstag und Bekanntmachung des Hinweises auf die Patenterteilung: **22.12.2004**
(21) Anmeldenummer: 99120759.8
(22) Anmeldetag: 20.10.1999
(51) Int. Cl.: C07D 217/02

(54) **Verfahren zur Reinigung von Isochinolin**
Process for the purification of isoquinoline
Procédé de purification d'isoquinoléine

(30) Priorität: 04.11.1998 DE 19850645
(43) Veröffentlichungstag der Anmeldung: 10.05.2000
(73) Patentinhaber: RÜTGERS Chemicals AG, 44579 Castrop-Rauxel (DE)
(72) Erfinder: Talbiersky, Jörg, Dr., 46282 Dorsten (DE); Fuhrmann, Edgar, Dr., 44579 Castrop-Rauxel (DE); Brüggemann, Wolfgang, 44581 Castrop-Rauxel (DE)
(74) Vertreter: Minderop, Ralph H., Dr. rer. nat.

(56) Entgegenhaltungen:
- US-A- 2 483 420

## Beschreibung

Die Erfindung betrifft ein Verfahren zur Reinigung von Isochinolin durch Kristallisation.

Isochinolin ist ein wichtiges Ausgangsmaterial zur Herstellung von Arzneimitteln und Sensibilisierungsfarbstoffen. Darüber hinaus findet Isochinolin als Lösungs- und Extraktionsmittel Verwendung. Reines Isochinolin bildet bei Raumtemperatur farblose Kristalle mit einem Schmelzpunkt von 26,5°C und besitzt bei Normaldruck einen Siedepunkt von 243,25°C.

Es ist bekannt, Isochinolin durch destillative Aufbereitung von Steinkohlenrohteer zu gewinnen, in dem es in einer Konzentration von etwa 0,06 Gew.% enthalten ist. Einschlägige Verfahren zur Gewinnung von Isochinolin aus Steinkohlenteer sind etwa in H.-G. Franck und G. Collin Steinkohlenteer", Springer-Verlag, Berlin, 1968, Seite 60 bis 64 beschrieben. Dabei geht man in der Regel von der entweder unmittelbar aus der kontinuierlichen Rohteerdestillation abgezogenen oder durch Aufarbeitung von Waschöl gewonnenen Methylnaphthalinfraktion mit einem Siedebereich von etwa 240 bis 245°C aus. Aus dieser wird durch Extraktion mit Schwefelsäure und anschließender Umsetzung der Basenschwefelsäure mit Ammoniakwasser Rohchinolin gewonnen, das etwa 55 Gew.% Chinolin (Sdp. 237,1°C), 13 Gew.% Isochinolin (Sdp. 243,2°C) und 8 Gew.% Chinaldin (Sdp. 247,6°C) enthält.

Bei der destillativen Aufarbeitung von Rohchinolin erhält man verschiedene Destillatfraktionen, darunter eine isochinolinreiche Fraktion mit einem Isochinolingehalt von etwa 70 Gew.% (Isochinolin 70"). Aus diesem Rohstoff kann durch fraktionierte Destillation Isochinolin mit einem Reinheitsgrad von bis zu 95% gewonnen werden. Höhere Isochinolinqualitäten können auf destillativem Weg nicht erzielt werden, da die Qualität durch Siedebegleiter limitiert wird. Es ist auch bekannt, das gewonnene Destillat durch Kristallisation auf einen Reinheitsgrad von bis zu 99,9% weiterzureinigen.

Aus der JP-01153679 (Chemical Abstracts 111:174007) ist bekannt, Isochinolin durch mehrfache Kristallisation aus Trialkylaminen als Lösungsmittel bis zu einem Reinheitsgrad von 99,9% mit einer Ausbeute von bis zu 62% zu reinigen. Nachteilig an diesem Verfahren ist, daß eine mehrfache Kristallisation des Isochinolins notwendig ist. Nachteilig ist ferner, daß das Verfahren eine Verwendung von Lösungsmitteln vorsieht, deren Reste nach der Kristallisation aus dem Endprodukt entfernt werden müssen.

In der JP-02049770 (Chemical Abstracts 113:6184) wird ein Verfahren zur Reinigung von Isochinolin vorgeschlagen, in dem 90%iges Isochinolin aus der Steinkohlenteeraufarbeitung zunächst mit Benzol als Lösungsmittel versetzt, auf -20°C abgekühlt und die gebildeten Kristalle bei 21°C mit Hexan gewaschen wurden. Das nach dem Verfahren in einer Ausbeute von 46% gewonnene Isochinolin weist einen Reinheitsgrad von 99,6% auf. Nachteilig an diesem Verfahren ist wiederum die Verwendung von Lösungsmitteln zur Kristallisation, deren Reste aus dem Endprodukt durch Abziehen entfernt werden müssen.

Aus der JP-03034970 (Chemical Abstracts 114:247160) ist ein Verfahren zur Reinigung von Isochinolin bekannt, in dem mindestens 90%iges Isochinolin mit Kupfer(II)-chlorid in Ethanol zu dem entsprechenden Kupferkomplex umgesetzt wird. Aus dem von der Mutterlauge abgetrennten und mit Ethanol gewaschenen Kupferkomplex wird durch Behandeln mit wässriger Alkalihydroxidlösung, Extrahieren mit Benzol und anschließendem Destillieren der organischen Phase 99,8%iges Isochinolin gewonnen. Auch dieses Verfahren umfaßt eine Anzahl von aufwendigen Verfahrensschritten und eignet sich daher nicht zur großtechnischen Herstellung von reinem Isochinolin.

Die US 2 483 420 betrifft die Reinigung einer durch Destillation erhaltenen Isochinolinfraktion mit einem Festpunkt von 8 bis 12°C durch Kühlen des destillierten Produkts bis unter 0°C, Aushärten des Isochinolins, Bilden einer halbflüssigen Masse und Extrahieren des festen Isochinolins aus dieser Mutterlauge.

Der Erfindung liegt die Aufgabe zugrunde, ein verbessertes Verfahren zur Reinigung von Isochinolin bereitzustellen, in dem ausgehend von mindestens 85%igem Isochinolin aus der Steinkohlenteeraufbereitung durch einmalige Kristallisation Isochinolin mit einer Reinheit von mindestens 97% erhalten werden kann. Insbesondere soll die Kristallisation auch ohne Verwendung von Lösungsmitteln durchführbar sein.

Diese Aufgabe wird dadurch gelöst, daß man in einer Isochinolinfraktion einen Chinolingehalt von bis zu etwa 1 Gew.% und einen Chinaldingehalt von bis zu etwa 8 Gew.% einstellt und diese Isochinolinfraktion einer Kristallisation unterzieht.

Erfindungsgemäß wurde festgestellt, daß die Kristallisation von Isochinolin besonders vorteilhaft verläuft, wenn das Ausgangsmaterial einen Chinolingehalt von weniger als etwa 1 Gew.% und einen Chinaldingehalt von weniger als etwa 8 Gew.% besitzt. Dieses Ergebnis war nicht offensichtlich, da man aufgrund der Schmelzpunkte der genannten Verunreinigungen (Chinolin: Smp. -14,2°C; Chinaldin: Smp. 1°C) ein umgekehrtes Verhalten erwarten würde.

Mit dem erfindungsgemäßen Verfahren ist es möglich, Isochinolin mit einem relativ geringen Reinheitsgrad (>85%) durch einen einzigen Verfahrensschritt auf einen Reinheitsgrad zu bringen, der eine Verwendung als Ausgangsmaterial zur Herstellung von Arzneimitteln erlaubt (>97%).

Bei der Steinkohlenteeraufarbeitung fällt eine isochinolinreiche Fraktion mit einem Isochinolingehalt von etwa 70 Gew.% an. Diese Isochinolin 70-Fraktion wird einer fraktionierten Destillation unterzogen, wodurch ein Isochinolingehalt von etwa 85 bis 95 Gew.% erzielt wird. Vorzugsweise wird die Destillation in einer mit einer Kolonne, insbesondere einer Füllkörperkolonne, ausgestatteten Apparatur durchgeführt. Die Destillation kann bei Normaldruck erfolgen. Vorzugsweise wird sie jedoch bei einem Kopfdruck von 100 bis 300 mbar, insbesondere 120 bis 180 mbar, durchgeführt. Am günstigsten hat sich ein Kopfdruck von etwa 150 mbar erwiesen. Vorzugsweise wird die Destillation bei einer Kopftemperatur von 165 bis 180°C, insbesondere 170 bis 175°C, durchgeführt. Das Rückflußverhältnis kann beliebig gewählt werden. Als besonders geeignet hat sich jedoch ein Rückflußverhältnis von etwa 1:20 erwiesen.

Nach der Destillation werden die gewonnenen Fraktionen durch geeignete Methoden wie Chromatographie, insbesondere Gaschromatographie, auf ihre Zusammensetzung untersucht. In der Regel weist der Hauptanteil der das Destillat ausmachenden Fraktionen (etwa 40 bis 50 Gew.%, bezogen auf die Menge des Ausgangsmaterials) einen Isochinolingehalt von etwa 95 Gew.% auf. Nur etwa 2 bis 4 Gew.% der Fraktionen, bezogen auf die Menge des Ausgangsmaterials, weisen einen Isochinolingehalt von mehr als 95 Gew.% auf.

Ein für das erfindungsgemäße Kristallisationsverfahren verwendbares Ausgangsmaterial kann dadurch bereitgestellt werden, daß man die bei der Destillation von Isochinolin 70 erhaltenen Fraktionen derart vereint, daß das Gemisch einen Chinolingehalt von höchstens 1 Gew.% und einen Chinaldingehalt von höchstens 8 Gew.% aufweist. In der Regel beträgt die Menge der vereinten Fraktionen, die diese Auflagen erfüllen, etwa 15 bis 20 Gew.%, bezogen auf die Menge des Ausgangsmaterials. Die vereinten Fraktionen weisen dabei einen durchschnittlichen Chinolingehalt von etwa 0,5 Gew.% und einen Chinaldingehalt von etwa 4 bis 5 Gew.% auf.

Die Kristallisation kann mit üblichen Kristallisationsverfahren, insbesondere durch Suspensionskristallisation, erfolgen. Die Suspensionskristallisation kann ein Abschleudern der Mutterlauge und/oder ein partielles Schmelzen umfassen. Vorzugsweise wird eine Suspensionskristallisation unter Abschleudern der Mutterlauge durchgeführt.

Es ist ferner möglich, der Suspension ein Verdünnungsmittel wie Toluol oder Arsol, beispielsweise Arsol VE zuzusetzen. Arsol VE ist ein leichtes Aromatengemisch, das als Hauptkomponenten Benzol, Toluol und Xylole enthält. In der Regel wird man jedoch auf den Zusatz von Verdünnungsmitteln verzichten, da dieser eine zusätzliche destillative Abtrennung von Lösungsmittelresten im Endprodukt erforderlich macht. Ein besonderer Vorteil des erfindungsgemäßen Verfahrens liegt darin, daß sich auch ohne Zusatz von Verdünnungsmitteln hervorragende Ausbeuten erzielen lassen.

Gemäß einer bevorzugten Ausführungsform der Erfindung werden die vereinten Isochinolin-Fraktionen aus der fraktionierten Destillation in einem geeigneten Gefäß, insbesondere in einem Doppelmantelgefäß, zunächst bis zur Kristallisation auf eine Temperatur von 5 bis 18°, insbesondere 11 bis 14°C gekühlt und bei dieser Temperatur 0,5 bis 3 Stunden, beispielsweise etwa 1 Stunde, gerührt. Anschließend wird der Kristallisationsbrei in einer vorgekühlten Zentrifuge scharf abgeschleudert. Das dabei erzeugte Isochinolin hat einen durchschnittlichen Gehalt von mehr als 97 Gew.%.

Das erfindungsgemäße Verfahren wird anhand der folgenden Beispiele weiter erläutert. Alle in den Tabellen 1 bis 5 enthaltenen Prozentangaben beziehen sich auf das Gewicht.

### Beispiel 1

### Erzeugung von Isochinolin 90 durch Destillation von Isochinolin 70

Isochinolin 70 wurde an einer 2 m Laborkolonne (Füllkörperkolonne) bei einem Kopfdruck von 150 mbar, einer Kopftemperatur von 170 bis 175°C und einem Rückflußverhältnis von 1:20 fraktionierend rektifiziert. Bei der Destillation wurden maximale Isochinolingehalte von 97,4 Gew.% erreicht. Die Ausbeute an Isochinolin 95 betrug 41,9% vom Einsatz bzw. 57,8%, bezogen auf Isochinolin im Einsatz.

Bei der Destillation wurden die in Tabelle 1 aufgeführten Fraktionen entnommen. Die in Tabelle 1 und den folgenden Tabellen verwendeten Abkürzungen "NSV", "n.i.P." und HSV" stehen für niedrig siedende Verbindungen", nicht identifizierbare Peaks" und "hochsiedende Verbindungen".

**Tabelle 1**

| Destillation von Isochinolin 70 | | | | | | | | | |
|---|---|---|---|---|---|---|---|---|---|
| | NSV % | Chinolin % | Isochinolin % | n.i.P. % | Chinaldin % | 8-Methyl-chinolin % | 3-Methyl-isochinolin % | Lepidin % | HSV % |
| Einsatz | 1,50 | 10,05 | 72,40 | 1,30 | 11,27 | 3,26 | 0,06 | 0,04 | 0,12 |

| Fraktion | | | | | | | | | |
|---|---|---|---|---|---|---|---|---|---|
| 1 | 100,00 | 0,00 | 0,00 | 0,00 | 0,00 | 0,00 | 0,00 | 0,00 | 0,00 |
| 2 | 48,01 | 41,88 | 8,87 | 0,71 | 0,06 | 0,43 | 0,01 | 0,00 | 0,03 |
| 3 | 1,26 | 72,14 | 24,09 | 1,24 | 0,14 | 1,04 | 0,00 | 0,00 | 0,09 |
| 4 | 0,30 | 63,92 | 34,34 | 0,81 | 0,07 | 0,56 | 0,00 | 0,00 | 0,00 |
| 5 | 0,34 | 58,38 | 37,97 | 1,48 | 0,09 | 1,63 | 0,00 | 0,00 | 0,11 |
| 6 | 0,18 | 36,88 | 59,20 | 1,47 | 0,12 | 2,00 | 0,00 | 0,00 | 0,15 |
| 7 | 0,12 | 26,73 | 69,53 | 1,40 | 0,20 | 1,90 | 0,00 | 0,00 | 0,12 |
| 8 | 0,09 | 16,34 | 79,96 | 1,53 | 0,17 | 1,76 | 0,00 | 0,00 | 0,15 |
| 9 | 0,09 | 13,12 | 83,11 | 1,57 | 0,27 | 1,72 | 0,00 | 0,00 | 0,12 |
| 10 | 0,01 | 6,25 | 90,88 | 1,28 | 0,23 | 1,34 | 0,00 | 0,00 | 0,01 |
| 11 | 0,07 | 5,30 | 91,29 | 1,50 | 0,32 | 1,42 | 0,00 | 0,00 | 0,10 |
| 12 | 0,08 | 2,75 | 94,32 | 1,26 | 0,28 | 1,19 | 0,00 | 0,00 | 0,12 |
| 13 | 0,04 | 1,38 | 95,79 | 1,18 | 0,40 | 1,16 | 0,00 | 0,00 | 0,05 |
| 14 | 0,10 | 0,25 | 97,41 | 0,86 | 0,36 | 0,94 | 0,00 | 0,00 | 0,08 |
| 15 | 0,14 | 0,53 | 96,44 | 1,30 | 0,53 | 0,71 | 0,00 | 0,00 | 0,35 |
| 16 | 0,54 | 0,23 | 96,45 | 1,29 | 0,53 | 0,88 | 0,00 | 0,00 | 0,08 |
| 17 | 0,71 | 0,12 | 96,07 | 1,19 | 0,86 | 1,00 | 0,00 | 0,00 | 0,05 |
| 18 | 1,68 | 0,08 | 95,22 | 1,09 | 0,85 | 0,99 | 0,00 | 0,00 | 0,09 |
| 19 | 1,47 | 0,02 | 95,09 | 1,40 | 0,94 | 1,04 | 0,00 | 0,00 | 0,04 |
| 20 | 2,28 | 0,03 | 94,23 | 1,22 | 1,04 | 1,18 | 0,00 | 0,00 | 0,02 |
| 21 | 1,87 | 0,02 | 93,58 | 1,27 | 1,75 | 1,48 | 0,00 | 0,00 | 0,03 |
| 22 | 2,07 | 0,00 | 94,29 | 0,40 | 1,63 | 1,59 | 0,00 | 0,00 | 0,02 |
| 23 | 2,71 | 0,00 | 91,59 | 0,40 | 2,93 | 2,35 | 0,00 | 0,00 | 0,02 |
| 24 | 0,56 | 0,00 | 90,52 | 0,63 | 4,59 | 3,67 | 0,00 | 0,00 | 0,03 |
| Dest. Ruckstand | 0,88 | 0,06 | 17,38 | 1,88 | 65,75 | 12,88 | 0,47 | 0,27 | 0,43 |

### Beispiel 2

### Kristallisation von Isochinolin

Eine aus Beispiel 1 erhaltene Isochinolinfraktion mit einem Gehalt von 91,8 Gew.% und der in Tabelle 2 wiedergegebenen Zusammensetzung wurde in einem Doppelmantelgefäß auf 13°C bis zur Kristallisation gekühlt und bei dieser Temperatur 1 Stunde lang langsam gerührt. Anschließend wurde der Kristallisationsbrei in einer vorgekühlten Zentrifuge scharf abgeschleudert (Schleuderöl: 52,2 Gew.%; Schleudergut: 43,1 Gew.%). Das Schleudergut wies einen Isochinolingehalt von 97,83 Gew.% auf. Die Ausbeute an gereinigtem Isochinolin betrug 43,1% vom Einsatz bzw. 45,9%, bezogen auf Isochinolin im Einsatz. Die übrige Zusammensetzung des Schleuderguts ist aus Tabelle 2 ersichtlich.

**Tabelle 2**

| | Einsatz Charge (%) | Schleudergut (%) |
|---|---|---|
| NSV | 0,43 | |
| Phenole | | 0,05 |
| Toluidine | | 0,03 |
| Xylidine | | 0,01 |
| Chinolin | 0,69 | 0,05 |
| Isochinolin | 91,80 | 97,83 |
| n.i.P. | 1,67 | 0,41 |
| Chinaldin | 4,08 | 1,30 |
| 8-Methylchinolin | 1,29 | 0,32 |
| 3-Methylisochinolin | 0,04 | |
| HSV | | |
| Summe | 100 | 100 |

### Beispiel 3

### Kristallisation von Isochinolin

Eine aus Beispiel 1 erhaltene Isochinolinfraktion mit einem Gehalt von 88,12 Gew.% und der in Tabelle 3 angegebenen Zusammensetzung wurde in einem Doppelmantelgefäß auf 12°C bis zur Kristallisation gekühlt und bei dieser Temperatur 1 Stunde lang langsam gerührt. Anschließend wurde der Kristallisationsbrei in einer vorgekühlten Zentrifuge scharf abgeschleudert (Schleuderöl: 49,9 Gew.%; Schleudergut: 47,2 Gew.%). Das Schleudergut wies ein Reinheit von 97,35% auf. Die Ausbeute an gereinigtem Isochinalin betrug 47,2% vom Einsatz bzw. 52,1%, bezogen auf Isochinolin im Einsatz. Die übrige Zusammensetzung des Schleuderguts ist aus Tabelle 3 ersichtlich.

**Tabelle 3**

| | Einsatz Charge (%) | Schleudergut (%) |
|---|---|---|
| NSV | | |
| Phenole | | |
| Toluidine | 0,41 | 0,07 |
| Xylidine | | |
| Chinolin | 0,33 | 0,18 |
| Isochinolin | 88,12 | 97,35 |
| n.i.P. | 1,34 | 0,38 |
| Chinaldin | 7,15 | 1,50 |
| 8-Methylchinolin | 2,65 | 0,52 |
| 3-Methylisochinolin | | |
| HSV | | |
| Summe | 100 | 100 |

### Beispiel 4

### Kristallisation von Isochinolin unter Zusatz von Verdünnungsmittel

Eine aus Beispiel 1 erhaltene Isochinolinfraktion mit einem Gehalt von 91,80 Gew.% und der in Tabelle 4 wiedergegebenen Zusammensetzung wurde mit 10 Gew.% Arsol VE Verdünnungsmittel, bezogen auf die Menge der Isochinolinfraktion, versetzt und in einem Doppelmantelgefäß auf 4°C bis zur Kristallisation gekühlt und bei dieser Temperatur 1 Stunde lang langsam gerührt. Anschließend wurde der Kristallisationsbrei in einer vorgekühlten Zentrifuge scharf abgeschleudert (Schleuderöl: 70,1 Gew.%; Schleudergut: 29,6 Gew.%) Das Schleudergut wies eine Reinheit von 96,21% auf. Die Ausbeute an gereinigtem Isochinolin betrug 29,6% vom Einsatz bzw. 34,13%, bezogen auf Isochinolin im Einsatz. Die übrige Zusammensetzung des Schleuderguts ist aus Tabelle 4 ersichtlich.

**Tabelle 4**

| | Einsatz Charge (%) | Schleudergut (%) |
|---|---|---|
| NSV | 0,43 | 1,98 |
| Phenole | | 0,05 |
| Toluidine | | 0,04 |
| Xylidine | | 0,02 |
| Chinolin | 0,69 | 0,05 |
| Isochinolin | 91,80 | 96,21 |
| n.i.P. | 1,67 | 0,34 |
| Chinaldin | 4,08 | 1,05 |
| 8-Methylchinolin | 1,29 | 0,26 |
| 3-Methylisochinolin | 0,04 | |
| HSV | | |
| Summe | 100 | 100 |

### Beispiel 5 (Vergleichsbeispiel)

### Kristallisation von chinolinreichem Isochinolin

Eine aus Beispiel 1 erhaltene Isochinolinfraktion mit einem Isochinolingehalt von 86,9 Gew.%, einem Chinolingehalt von 6,03 Gew.% und der in Tabelle 5 wiedergegebenen übrigen Zusammensetzung wurde in einem Doppelmantelgefäß auf 13°C bis zur Kristallisation gekühlt und bei dieser Temperatur 1 Stunde lang langsam gerührt. Anschließend wurde der Kristallisationsbrei in einer vorgekühlten Zentrifuge scharf abgeschleudert (Schleuderöl: 53 Gew.%; Schleudergut: 46,7 Gew.%). Die Ausbeute an gereinigtem Isochinolin betrug 46,7% vom Einsatz bzw. 51,2%, bezogen auf Isochinolin im Einsatz. Das erhaltene Schleudergut wies eine Reinheit von 95,29% auf. Die übrige Zusammensetzung des Schleuderguts ist aus Tabelle 5 ersichtlich.

**Tabelle 5**

| | Einsatz Charge (%) | Schleudergut (%) |
|---|---|---|
| NSV | 0,02 | |
| Phenole | 0,10 | 0,01 |
| Toluidine | 0,38 | 0,07 |
| Xylidine | 0,03 | 0,02 |
| Chinolin | 6,03 | 3,13 |
| Isochinolin | 86,90 | 95,29 |
| n.i.P. | 1,32 | 0,36 |
| Chinaldin | 3,19 | 0,72 |
| 8-Methylchinolin | 1,95 | 0,40 |
| 3-Methylisochinolin | | |
| HSV | 0,08 | |
| Summe | 100 | 100 |

## Patentansprüche

1. Verfahren zur Reinigung von Isochinolin, **dadurch gekennzeichnet, daß** eine Isochinolinfraktion aus der Steinkohlenteeraufarbeitung mit einem Reinheitsgrad von mindestens 85 % und mit einem Chinolingehalt von bis zu etwa 1 Gew.% und einem Chinaldingehalt von bis zu etwa 8 Gew.% einer Kristallisation unterzogen wird.

2. Verfahren gemäß Anspruch 1, **dadurch gekennzeichnet, daß** die Kristallisation eine Suspensionskristallisation ist.

3. Verfahren gemäß Anspruch 2, **dadurch gekennzeichnet, daß** die Suspensionskristallisation unter Abschleudern der Mutterlauge erfolgt.

4. Verfahren gemäß Anspruch 2 oder 3, **dadurch gekennzeichnet, daß** die Kristallisation durch Abkühlen der Isochinolinfraktion auf eine Temperatur von 5 bis 18°C, insbesondere 11 bis 14°C, eingeleitet wird.

## Claims

1. A process for purifying isoquinoline, **characterized in that** an isoquinoline fraction from the treatment of bituminous coal tar having a degree of purity of at least 85 % and a quinoline content of up to approximately 1 % by weight and a quinaldine content of up to approximately 8 % by weight is subjected to crystallization.

2. A process as claimed in claim 1, **characterized in that** crystallization is a suspension crystallization.

3. A process as claimed in claim 2, **characterized in that** the suspension crystallization occurs by centrifuging of the mother liquor.

4. A process as claimed in claim 2 or 3, **characterized in that** crystallization is introduced by cooling of the isoquinoline fraction to a temperature of 5 to 18 °C, in particular 11 to 14 °C.

## Revendications

1. Procédé de purification d'isoquinoléine, **caractérisé en ce qu'**une fraction d'isoquinoléine provenant du traitement du goudron de houille, avec un taux de pureté d'au moins 85% et avec une teneur en quinoléine de jusqu'à environ 1% en poids, et une teneur en quinaldine de jusqu'à environ 8% en poids, est soumise à cristallisation.

2. Procédé selon la revendication 1, **caractérisé en ce que** la cristallisation est une cristallisation en suspension.

3. Procédé selon la revendication 2, **caractérisé en ce que** la cristallisation en suspension est réalisée avec essorage des eaux mères.

4. Procédé selon la revendication 2 ou 3, **caractérisé en ce que** la cristallisation est déclenchée par refroidissement de la fraction d'isoquinoléine jusqu'à une température allant de 5 à 18°C, en particulier de 11 à 14°C.
